(19) Europäisches Patentamt
European Patent Office
Office européen des brevets

(11) Veröffentlichungsnummer: **0 011 179**
**B1**

# (12) EUROPÄISCHE PATENTSCHRIFT

(45) Veröffentlichungstag der Patentschrift: **15.02.84**

(51) Int. Cl.³: **C 07 D 319/20**, C 07 D 317/46, C 07 D 405/12, A 01 N 47/36

(21) Anmeldenummer: **79104215.3**

(22) Anmeldetag: **30.10.79**

## (54) N-Phenylharnstoffe, Verfahren zu ihrer Herstellung, ihre Verwendung als Herbizide, sowie Zwischenprodukte und deren Herstellung.

(30) Priorität: **09.11.78 DE 2848531**

(43) Veröffentlichungstag der Anmeldung: **28.05.80 Patentblatt 80/11**

(45) Bekanntmachung des Hinweises auf die Patenterteilung: **15.02.84 Patentblatt 84/7**

(84) Benannte Vertragsstaaten: **BE CH DE FR GB IT NL**

(56) Entgegenhaltungen: **DE - A - 2 624 822**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Lantzsch, Reinhard, Dr., Heymannstrasse 32, D-5090 Leverkusen (DE)**
Erfinder: **Marhold, Albrecht, Dr., Carl-Duisberg-Strasse 329, D-5090 Leverkusen (DE)**
Erfinder: **Eue, Ludwig, Dr., Paul-Klee-Strasse 36, D-5090 Leverkusen (DE)**
Erfinder: **Schmidt, Robert Rudolf, Dr., Hahnenweg 5, D-5000 Köln 80 (DE)**

EP 0 011 179 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

N-Phenylharnstoffe, Verfahren zu ihrer Herstellung, ihre Verwendung als Herbizide, sowie Zwischenprodukte und deren Herstellung

Die Erfindung betrifft neue, fluorhaltige N-Phenylharnstoffe, mehrere Verfahren zu ihrer Herstellung sowie ihre Verwendung als Herbizide, sowie Zwischenprodukte und deren Herstellung.

Es ist bereits bekannt, dass bestimmte N-Phenylharnstoffe, die Sauerstoffheterocyclen enthalten, als Herbizide Verwendung finden können, so z.B. 1-(2,2-Dimethyl-1,3-benzodioxol-5-yl)-3,3-dimethylharnstoff (vgl. DE-OS2624822). Diese bekannten Harnstoffe befriedigen jedoch in ihrer Unkrautwirkung nicht immer.

Es wurden nun neue N-Phenylharnstoffe der allgemeinen Formel I

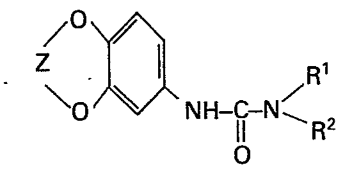

(I),

in welcher

Z für $-CF_2-$, $-CF_2CH_2-$, $-CF_2CHF-$ oder $-CF_2CFCl-$ steht,

$R^1$ für Wasserstoff, $C_{1-4}$-Alkyl, $C_{3-5}$-Alkenyl oder $C_{3-5}$-Alkinyl steht und

$R^2$ für $C_{1-4}$-Alkyl, $C_{3-5}$-Alkenyl, $C_{3-5}$-Alkinyl oder $C_{1-4}$-Alkoxy steht oder

$R^1$ und $R^2$ zusammen mit dem benachbarten N-Atom einen heterocyclischen 5- oder 6-Ring bilden,

aufgefunden.

Man erhält die N-Phenylharnstoffe der allgemeinen Formel I, wenn man

(a) Isocyanate der allgemeinen Formel II

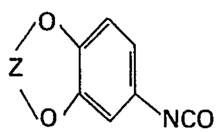

(II),

in welcher

Z die oben angegebene Bedeutung hat,

mit Aminen der allgemeinen Formel III

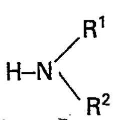

(III),

in welcher

$R^1$ und $R^2$ die oben angegebene Bedeutung haben,

gegebenenfalls in Gegenwart eines Verdünnungsmittels umsetzt oder

(b) für den Fall, dass $R^2$ für $C_{1-4}$-Alkoxy steht, Isocyanate der allgemeinen Formel II mit Hydroxylamin ($NH_2OH$) zu neuen Hydroxyharnstoffen der allgemeinen Formel IV umsetzt

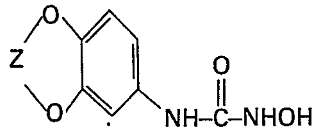

(IV),

in welcher

Z die oben angegebene Bedeutung hat,

und diese anschliessend mit einem Alkylierungsmittel der allgemeinen Formel V

$$X\text{-}R^3 \qquad (V),$$

in welcher

$R^3$ für $C_{1-4}$-Alkyl steht und

X für Halogen oder $R^3SO_4$ steht,

in Gegenwart einer Base und gegebenenfalls in Gegenwart eines Verdünnungsmittels umsetzt oder

(c) für den Fall, dass $R^1$ für Wasserstoff und $R^2$ für $C_{1-4}$-Alkyl oder $C_{3-5}$-Alkenyl stehen, Aniline der allgemeinen Formel VI

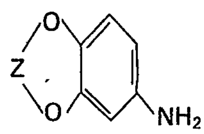

(VI),

in welcher

Z die oben angegebene Bedeutung hat,

mit Isocyanaten der allgemeinen Formel VII

$$R^4\text{–}NCO \qquad (VII),$$

in welcher

$R^4$ für $C_{1-4}$-Alkyl oder $C_{3-5}$-Alkenyl steht,

gegebenenfalls in Gegenwart eines Verdünnungsmittels umsetzt.

Die neuen N-Phenylharnstoffe der allgemeinen Formel I weisen starke herbizide Eigenschaften auf.

Überraschenderweise zeigen die erfindungsgemässen N-Phenylharnstoffe eine höhere herbizide Aktivität und insbesondere eine bessere Selektivität als die chemisch nächstliegenden bekannten Wirkstoffe gleicher Wirkungsart.

Von den erfindungsgemässen Verbindungen der allgemeinen Formel I sind diejenigen bevorzugt, in denen $R^1$ für Wasserstoff oder $C_{1-4}$-Alkyl und $R^2$ für $C_{1-4}$-Alkyl, $C_{3-5}$-Alkinyl- oder $C_{1-4}$-Alkoxy steht und in denen $R^1$ und $R^2$ zusammen mit dem benachbarten Stickstoffatom einen Pyrrolidin- oder Piperidinring bilden.

Ganz besonders bevorzugt sind die Verbindungen der allgemeinen Formel I, in denen $R^1$ für Wasserstoff oder Methyl steht und $R^2$ für Methyl oder Methoxy steht.

Verwendet man beispielsweise 2,2,3-Trifluorbenzodioxen-6-yl-isocyanat und Dimethylamin als Ausgangsstoffe, so kann der Reaktionsablauf gemäss Verfahren (a) durch das folgende Formelschema wiedergegeben werden:

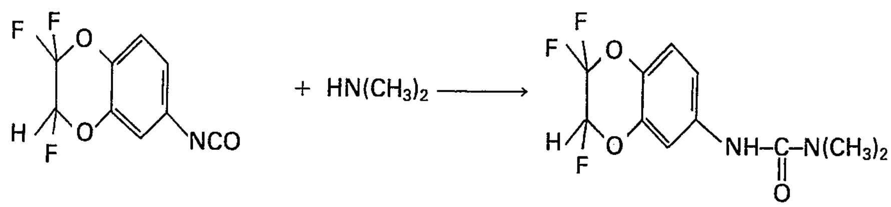


Verwendet man beispielsweise 2,2-Difluorbenzodioxol-5-yl-isocyanat und Hydroxylamin als Ausgangsstoffe und Dimethylsulfat als Alkylierungsmittel, so kann der Reaktionsablauf gemäss Verfahrensvariante (b) durch das folgende Formelschema wiedergegeben werden:

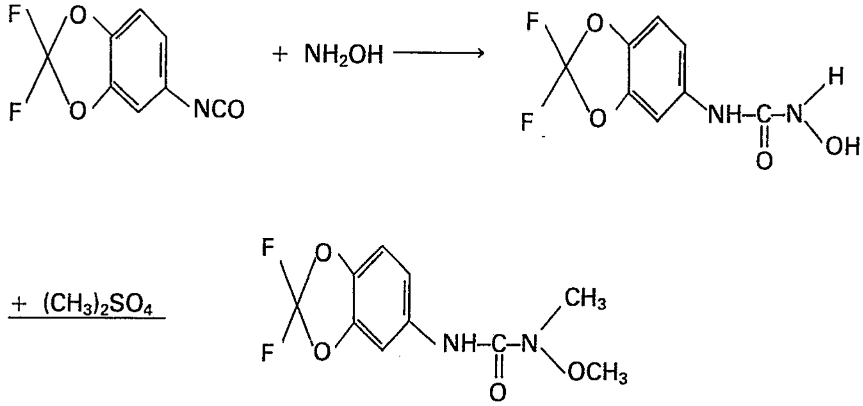


Verwendet man z.B. 6-Amino-2,2,3-trifluor-3-chlorbenzodioxen-(1,4) und Äthylisocyanat als Ausgangsstoffe, so kann der Reaktionsablauf gemäss Verfahrensvariante (c) durch das folgende Formelschema wiedergegeben werden:

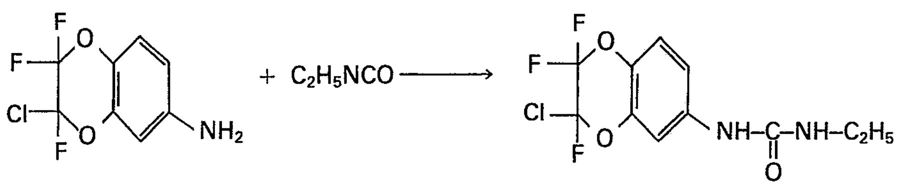


Die bei den Verfahrensvarianten (a) und (b) als Ausgangsstoffe zu verwendenden Isocyanate sind durch die allgemeine Formel II allgemein definiert. Es handelt sich hierbei im einzelnen um folgende Verbindungen:

2,2-Difluor-benzodioxol-5-yl-osocyanat.
2,2-Difluor-benzo-1,4-dioxen-6-yl-isocyanat,
2,2,3-Trifluor-benzo-1,4-dioxen-6-yl-isocyanat und
2,2,3-Trifluor-3-chlor-benzo-1,4-dioxen-6-yl-isocyanat.

Die Isocyanate der allgemeinen Formel II sind bisher noch nicht bekannt, können aber hergestellt werden, indem man in einer ersten Stufe Verbindungen der allgemeinen Formel VIII

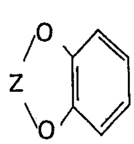 (VIII),

worin

Z die angegebene Bedeutung hat,

in an sich bekannter Weise nitriert, in einer zweiten Stufe die dadurch erhaltenen Nitroverbindungen der allgemeinen Formel IX

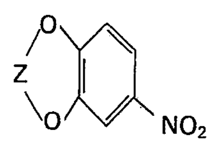 (IX),

worin

Z die angegebene Bedeutung hat,

in an sich bekannter Weise reduziert, vorzugsweise durch katalytische Hydrierung mit Wasserstoff, und in einer dritten Stufe die dadurch erhaltenen Aniline der allgemeinen Formel VI

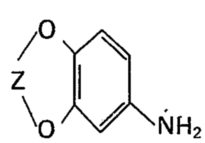 (VI)

worin
Z die angegebene Bedeutung hat,
in an sich bekannter Weise phosgeniert.

Die Verbindungen der allgemeinen Formel (VIIIa), (IXa) und (VIa)

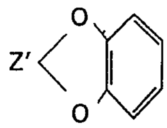

(VIIIa)

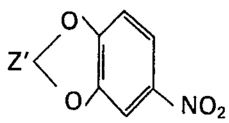

(IXa)

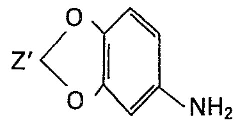

(VIa)

in welchen jeweils
Z' für $-CF_2-$ steht,
sind bekannt [Zh. Obsh. Khim. 34, 307–317 (1964), (VIIIa) und (IXa) Zh. Obsh. Khim. 31, 628–632 (1961), (VIa)]. Diese Verbindungen können auf folgendem Weg, ausgehend von Brenzkatechin, hergestellt werden:

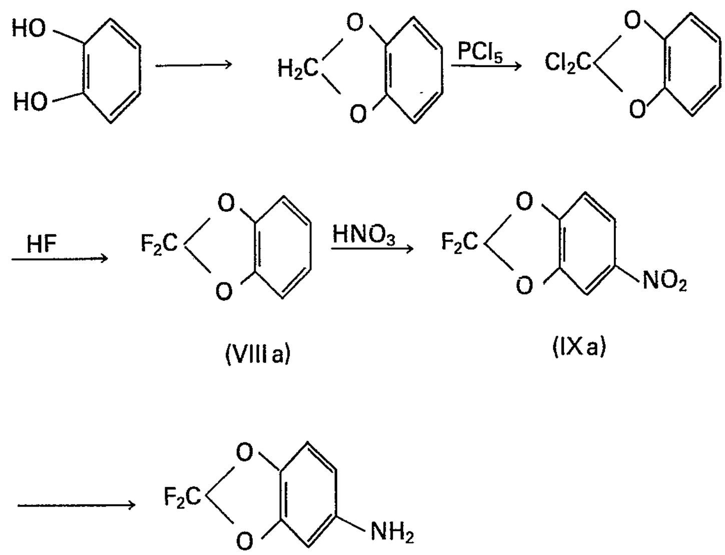


(VIa)

Die Verbindungen der allgemeinen Formel VIIIb

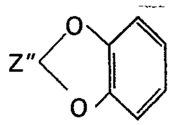

(VIIIb),

worin
Z" für $-CF_2CH_2-$ oder $-CF_2CHF-$ steht,
sowie das nachfolgend angegebene Verfahren zu ihrer Herstellung sind Gegenstand eines eigenen älteren Schutzbegehrens (Deutsche Patentanmeldung P28197888 vom 5.5.1978). Die Verbindungen der allgemeinen Formel VIIIb, d.h. die Verbindungen 2,2,-Difluor-benzodioxen(1,4) und 2,2,3-Trifluor-benzodioxen(1,4), können hergestellt werden, indem man Brenzkatechin

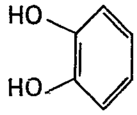

in Gegenwart von Basen mit einem Fluoräthylen der allgemeinen Formel X

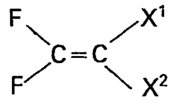

(X),

in welcher
$X^1$ für Halogen (insbesondere Fluor oder Chlor) steht und
$X^2$ für Wasserstoff oder Halogen (insbesondere Fluor oder Chlor) steht,
umsetzt.

Als Beispiele für Verbindungen der allgemeinen Formel X seien im einzelnen genannt:

Chlortrifluoräthylen, Bromtrifluoräthylen, Chlordifluoräthylen, Bromdifluoräthylen. Besonders bevorzugt sind Chlortrifluoräthylen und Chlordifluoräthylen.

Als Basen sind bei dieser Umsetzung besonders die Hydroxide der Alkali- und Erdalkalimetalle geeignet, ebenso aber auch die Carbonate dieser Metalle. Ihre Menge kann zwischen einem und mehr als drei Mol pro Mol Brenzkatechin schwanken. Ein Überschuss wirkt sich günstig aus. Die Umsetzung kann bei Temperaturen von 20 bis 150°C erfolgen, besonders bevorzugt im Bereich von 80 bis 120°C. Als Lösungsmittel werden polare Flüssigkeiten verwendet. Es haben sich beispielsweise Dimethylsulfoxid, Dimethylformamid und Tetramethylensulfon, aber auch Äther wie Dioxan oder Diglyme bewährt. Bevorzugt ist Tetramethylensulfon.

Die Verbindungen der allgemeinen Formel VIIIe

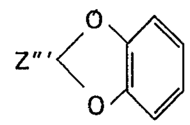

(VIIIe),

worin
Z''' für -$CF_2CFCl$- steht,
d.h. die Verbindung 2,2,3-Trifluor-3-chlor-benzodioxen(1,4) ist neu. Sie kann durch Chlorierung von 2,2,3-Trifluorbenzodioxen(1,4) bei Temperaturen zwischen etwa 60 und 150°C, vorzugsweise zwischen etwa 70 und 130°C, unter radikalischen Bedingungen hergestellt werden (vgl. Herstellungsbeispiele E).

Die Nitrierung der Verbindungen der allgemeinen Formel VIII wird in an sich bekannter Weise durchgeführt. Als Nitrierungsmittel wird Salpetersäure oder vorzugsweise «Nitriersäure» (Salpetersäure/Schwefelsäure) verwendet. Als Verdünnungsmittel können alle bei Nitrierungen üblichen organischen Lösungsmittel eingesetzt werden; vorzugsweise wird jedoch Wasser verwendet (vgl. Herstellungsbeispiele D).

Die Nitroverbindungen der allgemeinen Formel IXb

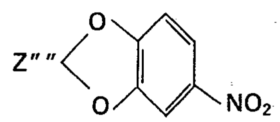

(IXb),

worin
Z'''' für -$CF_2CH_2$-, -$CF_2CHF$- oder -$CF_2CFCl$- steht, sind neu.

Die Reduktion der Nitroverbindungen der allgemeinen Formel IX zu Anilinen wird ebenfalls in an sich bekannter Weise durchgeführt (vgl. z.B. Houben-Weyl, Handbuch der Organischen Chemie, Band XI, 1, S.360–472). Bevorzugt erfolgt die Reduktion durch katalytische Hydrierung mittels Wasserstoff (vgl. Herstellungsbeispiele C).

Die Aniline der allgemeinen Formel VIb

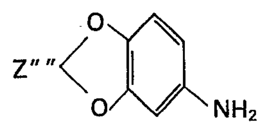

(VIb),

worin
Z'''' für -$CF_2CH_2$-, -$CF_2CHF$- oder -$CF_2CFCl$- steht, sind ebenfalls neu.

Die Phosgenierung der Aniline der allgemeinen Formel VI zu Isocyanaten und die Aufarbeitung erfolgt gleichfalls in an sich bekannter Weise (vgl. Herstellungsbeispiele B).

Die so erhaltenen Isocyanate der allgemeinen Formel II sind neu.

Die bei der Verfahrensvariante (a) weiterhin als Ausgangsstoffe zu verwendenden Amine der allgemeinen Formel III sind allgemein bekannt. Als Beispiele seien im einzelnen genannt:

Methylamin, Dimethylamin, Methylbutylamin, Diisopropylamin, Pyrrolidin, Piperidin, N,O-Dimethylhydroxylamin, Methylbutinylamin.

Die Alkylierungsmittel der allgemeinen Formel V sind ebenfalls allgemein bekannt. Als Beispiele seien genannt:

Methylchlorid, Methylbromid, Methyljodid, Äthylbromid, Äthyljodid, n- und iso-Propyljodid, n-Butylbromid, Dimethylfulat und Diäthylsulfat.

Die weiterhin als Ausgangsstoffe einzusetzenden Isocyanate der allgemeinen Formel VII sind gleichfalls allgemein bekannt. Als Beispiele seien angeführt:

Methylisocyanat, Äthylisocyanat, n- und iso-Propylisocyanat, n-Butylisocyanat, Allylisocyanat.

Als Verdünnungsmittel kommen bei Verfahren (a) alle inerten organischen Lösungsmittel in Frage, beispielsweise Kohlenwasserstoffe, wie Toluol, Xylol, Benzin, Petroläther, oder Chlorkohlenwasserstoffe wie Methylenchlorid, Chloroform, Chlorbenzol, ferner Äther wie Diäthyläther, Diisopropyläther oder Tetrahydrofuran.

Die Reaktionstemperaturen können bei Verfahren (a) in einem grösseren Bereich variiert werden. Im allgemeinen arbeitet man zwischen etwa 10 und 100°C, vorzugsweise zwischen etwa 20 und 60°C.

Bei der Durchführung des Verfahrens (a) setzt man die Reaktionskomponenten zweckmässigerweise in äquimolaren Mengen ein, jedoch schadet ein Aminüberschuss nicht. Im allgemeinen kann das Reaktionsprodukt direkt durch Abfiltrieren rein erhalten werden. Gegebenenfalls wird es durch Abdestillieren des Lösungsmittels isoliert und durch Umkristallisieren weiter gereinigt.

Die Herstellung der erfindungsgemässen Verbindungen der allgemeinen Formel I nach Verfahren (b) wird im allgemeinen in zwei Stufen durchgeführt, kann aber auch in einer Eintopfreaktion durchgeführt werden.

Im allgemeinen wird in der ersten Stufe gemäss Verfahren (b) Hydroxylamin in Gegenwart eines organischen Lösungsmittels, vorzugsweise jedoch in wässriger Lösung vorgelegt. Als organische Lösungsmittel kommen die beim Verfahren (a) genannten in Frage. Anschliessend wird das Isocyanat der allgemeinen Formel II, gegebenenfalls in einem der beim Verfahren (a) genannten Verdünnungsmittel, zugetropft. Die Reaktionstemperatur liegt bei der ersten Stufe zwischen etwa –10 und 30°C, vorzugsweise zwischen 0 und 20°C.

Die so entstandenen Hydroxyharnstoffe der allgemeinen Formel IV können direkt weiter alkyliert werden oder durch zugeben von Wasser ausgefällt und abfiltriert werden.

Auch die zweite Stufe des Verfahrens (b) wird vorzugsweise in Gegenwart eines Verdünnungsmittels durchgeführt. Zusätzlich zu den bei Verfahren (a) genannten Verdünnungsmitteln kommen auch Alkohole, wie Methanol oder Äthanol in Betracht.

Zur Durchführung der Alkylierungsreaktion wird eine Base, wie beispielsweise Natrium- oder Kaliumhydroxid, benötigt. Sie wird im allgemeinen äquimolar zum Alkylierungsmittel eingesetzt, von dem mindestens die doppelt äquimolare Menge, bezogen auf die Verbindung der allgemeinen Formel IV, benötigt wird.

Die Temperatur beträgt bei der zweiten Stufe des Verfahrens (b) zwischen 10 und 150°C, vorzugsweise zwischen 20 und 115°C, je nach Art und Reaktivität des Alkylierungsmittels.

Zur Beschleunigung der Alkylierungsreaktion kann gegebenenfalls in Gegenwart eines Phasentransferkatalysators gearbeitet werden. Als Phasentransferkatalysatoren kommen Kronenäther, quartäre Phosphonium- oder vorzugsweise quartäre Ammoniumsalze, wie beispielsweise Tetrabutylammoniumchlorid oder -bromid, Triäthylbenzylammoniumchlorid oder Methyltrioctylammoniumchlorid in Frage.

Die bisalkylierten Harnstoffe der allgemeinen Formel I werden in üblicher Weise durch Extraktion mit einem organischen Lösungsmittel aus dem Reaktionsgemisch abgetrennt. Nach Abdestillieren des Lösungsmittels werden die Harnstoffe gegebenenfalls durch Umkristallisieren gereinigt.

Das erfindungsgemässe Verfahren (c) wird in prinzipiell der gleichen Weise durchgeführt wie Verfahren (a). Hier gelten bezüglich Verdünnungsmittel, Temperaturbereich und Molverhältnissen die oben für Verfahren (a) gemachten Angaben. Die Herstellung der als Ausgangsprodukte benötigten Aniline der allgemeinen Formel VI ist oben bereits beschrieben worden (vgl. Herstellungsbeispiele C).

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dicotyle Kulturen der Gattungen:* Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Sphenoclea, Dactyloctenium, Agrostis, Alopecurus, Apera.

*Monokotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfingungsgemässen Wirkstoffe eignen sich vorzugsweise zur selektiven Unkrautbekämpfung in verschiedenen Kulturen, besonders in Getreide, Baumwolle und Mais.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewandt werden. Die Anwendung geschieht in üblicher Weise, z.B. durch Giessen, Spritzen, Sprühen, Stäuben oder Streuen.

Die erfindungsgemässen Wirkstoffe können sowohl nach als auch insbesondere vor Auflaufen der Pflanzen appliziert werden. Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

*Herstellungsbeispiele*

A) N-Phenylharnstoffe der allgemeinen Formel I

Beispiel (I)-1 [nach Verfahren (c)]

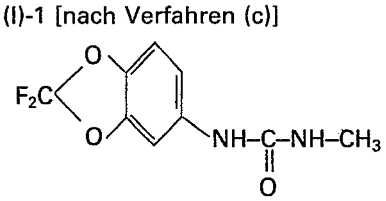

Man löst 17,3 g (0,1 Mol) 5-Amino-2,2-difluorbenzodioxol in 100 ml Methylenchlorid und tropft bei Raumtemperatur 5,7 g (0,1 Mol) Methylisocyanat in 50 ml Methylenchlorid zu. Man rührt noch 1 Stunde bei Raumtemperatur nach, filtriert das gebildete Produkt ab und trocknet es. Der so erhaltene 1-Methyl-3-(2,2-difluorbenzodioxol-5-yl)-harnstoff schmilzt bei 160 bis 161°C; Ausbeute 20,4 g (88,8% d.Th.).

In analoger Weise werden die folgenden Harnstoffe erhalten [nach Verfahren (c)]:

Beispiel Strukturformel Schmelzpunkt

(I)-2 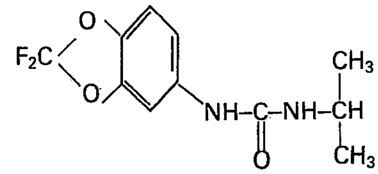 163°C

(I)-3 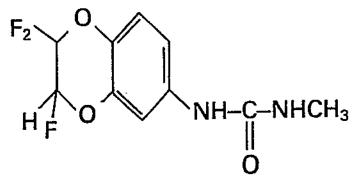 145°C

(I)-4 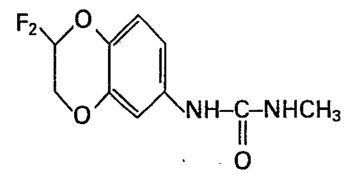 167°C

Beispiel (I)-5 [nach Verfahren (a)]

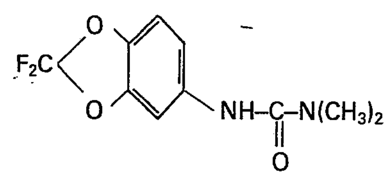

Man löst 19,9 g (0,1 Mol) 2,2-Difluor-benzodioxol-5-yl-isocyanat in 100 ml Toluol und leitet so lange gasförmiges Dimethylamin ein, bis im IR-Spektrum keine Isocyanatbande mehr zu sehen ist. Dann filtriert man das gebildete Produkt ab und kristallisiert es aus Äthanol um.

Man erhält 18,2 g 1,1-Dimethyl-3-(2,2-difluorbenzodioxol-5-yl)-harnstoff (75% d.Th.); Schmelzpunkt 130°C.

In analoger Weise werden die folgenden Harnstoffe erhalten [nach Verfahren (a)]:

Beispiel Strukturformel Schmelzpunkt

(I)-6 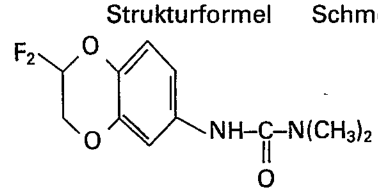 179°C

(I)-7 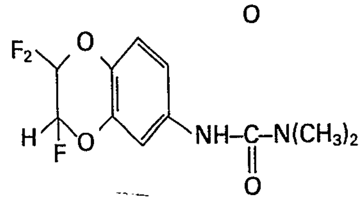 153°C

(I)-8 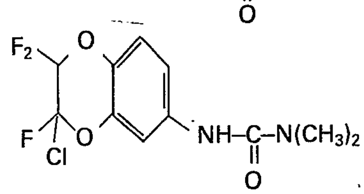 120°C

Beispiel (I)-9 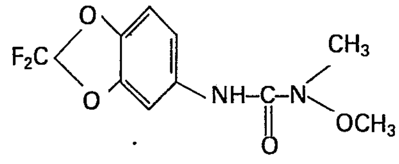

[nach Verfahren (a)]:

Man löst 19,9 g (0,1 Mol) 2,2-Difluor-benzodioxol-5-yl-isocyanat in 100 ml Methylenchlorid und tropft 6,1 g (0,1 Mol) N,O-Dimethylhydroxylamin, gelöst in 50 ml Methylenchlorid, bei Raumtemperatur hinzu. Man rührt noch 1 Stunde nach, destilliert das Methylenchlorid im Vakuum ab und kristallisiert den Rückstand aus Cyclohexan um.

Man erhält 20,3 g 1-Methyl-1-methoxy-3-(2,2-difluorbenzodioxol-5-yl)-harnstoff (78,3% d.Th.); Schmelzpunkt 66°C.

[nach Verfahren (b)]:

Man löst 7,0 g (0,1 Mol) Hydroxylaminchlorid und 4 g Natriumhydroxid in 20 ml Wasser und tropft bei 10 bis 15°C eine Lösung von 19,9 g (0,1 Mol) 2,2-Difluor-benzodioxol-5-yl-isocyanat in 100 ml Methylenchlorid zu. Man rührt 15 Minuten nach, gibt 1 g Tetrabutylammoniumbromid zu, sowie 20,9 ml Dimethylsulfat. Dann tropft man bei Raumtemperatur langsam 22 g 40%ige Natronlauge zu, rührt noch 2 Stunden nach, trennt die organische Phase ab, wäscht mit 50 ml Wasser, trocknet mit Natriumsulfat, filtriert ab und dampft das Filtrat am Rotationsverdampfer ein. Der zurückbleibende 1-Methyl-1-methoxy-3-(2,2-difluorbenzodioxol-5-yl)-harnstoff wird durch zweimaliges Umkristallisieren aus Cyclohexan gereinigt; Ausbeute 17,5 g (67,3% d. Th.); Schmelzpunkt 66°C.

In analoger Weise werden die folgenden Harnstoffe erhalten [nach Verfahren (a) und (b)]:

| Beispiel | Strukturformel | Schmelzpunkt |
|---|---|---|
| (I)-11 | 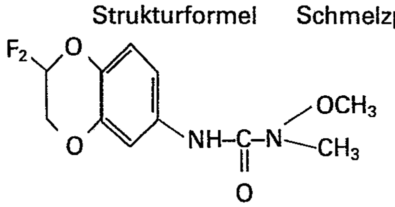 | 68°C |
| (I)-12 | 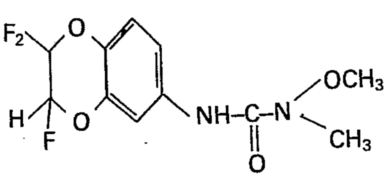 | 73°C |
| (I)-13 | 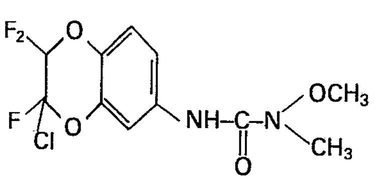 | 72°C |

Beispiel (I)-14 [nach Verfahren (a)]

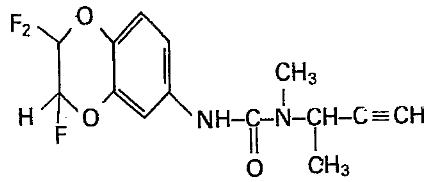

Man löst 23,1 g (0,1 Mol) 2,2,3-Trifluor-benzo-1,4-dioxen-6-yl-isocyanat in 100 ml Methylenchlorid und tropft 8,3 g (0,1 Mol) N-Methyl-1-methylpropinylamin in 50 ml Methylenchlorid bei Raumtemperatur zu. Man rührt noch 1 Stunde nach und erhält nach Abdestillieren des Methylenchlorids in quantitativer Ausbeute 1-Methyl-1-(1-methylpropinyl)-3-(2,2,3-trifluorbenzodioxen(1,4)-6-yl)-harnstoff; Schmelzpunkt 132°C.

Beispiel (I)-15 [nach Verfahren (a)]

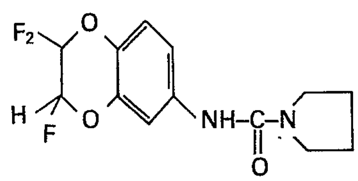

Analog zu Beispiel (I)-14 erhält man aus 23,1 g (0,1 Mol) 2,2,3-Trifluor-benzo-1,4-dioxen-6-yl-isocyanat und 7,1 g (0,1 Mol) Pyrrolidin den 1,1-Tetramethylen-3-(2,2,3-trifluor-benzodioxen-(1,4)-6-yl)-harnstoff, vom Schmelzpunkt 144°C; Ausbeute 30 g (99% d.Th.).

B) Isocyanate der allgemeinen Formel II

Beispiel (II)-1

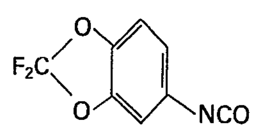

Man leitet bei 0°C 50 g Phosgen in 200 ml Chlorbenzol ein. Dann werden bei 0 bis 5°C 34,6 g 2,2-Difluor-5-amino-benzodioxol in 40 ml Chlorbenzol zugetropft. Man lässt auf Raumtemperatur kommen und erhitzt dann unter weiterem Einleiten von Phosgen 2 bis 3 Stunden zum Sieden. Anschliessend wird noch eine weitere Stunde ohne Einleiten von Phosgen gekocht und dann das überschüssige Phosgen mit Stickstoff ausgeblasen. Durch fraktionierte Destillation im Vakuum erhält man das 2,2-Difluor-benzodioxol-5-yl-isocyanat vom Siedepunkt 90°C/20 mbar. Brechungsindex $n_D^{20}$ = 1,495.

In analoger Weise werden die folgenden Isocyanate erhalten:

| Beispiel | Strukturformel | Siedepunkt | Brechungsindex $n_D^{20}$ |
|---|---|---|---|
| (II)-2 | 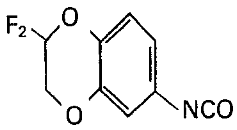 | 60°C/0,4 mbar | 1,515 |
| (II)-3 | 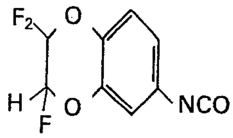 | 52°C/0,53 mbar | 1,495 |
| (II)-4 | 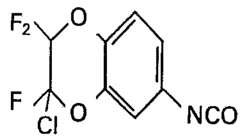 | 65–68°C/0,4 mbar | 1,497 |

C) Amine der allgemeinen Formel VIb

Beispiel (VIb)-1

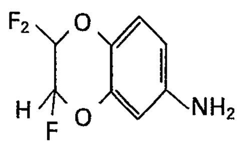

198 g 6-Nitro-2,2,3-trifluor-benzodioxen(1,4) werden in 600 ml Äthanol gelöst und 20 g Raney-Nickel werden zugegeben. Unter Rühren wird Wasserstoff mit 50 bar aufgedrückt bis Sättigung bei 45°C Innentemperatur erreicht ist. Nach Entspannen wird der Katalysator abfiltriert und das Filtrat destilliert. Man erhält 142 g 6-Amino-2,2,3-trifluor-benzodioxen(1,4) vom Siedepunkt 125 bis 127°C/21 mbar; $n_D^{20}$ = 1,501.

In analoger Weise werden die folgenden Amine erhalten:

| Beispiel | Strukturformel | Siedepunkt | Brechungsindex $n_D^{20}$ |
|---|---|---|---|
| (VIb)-2 | 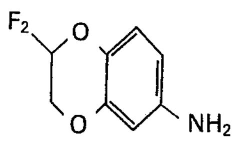 | 130–131°C/17,3 mbar | |
| (VIb)-3 | 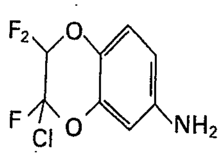 | 120–121°C/17,3 mbar | 1,5015 |

D) Nitroverbindungen der allgemeinen Formel IXb:

Beispiel (IXb)-1

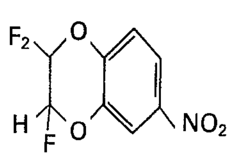

190 g 2,2,3-Trifluorbenzodioxen(1,4) werden bei 5°C vorgelegt; bei dieser Temperatur wird eine Mischung aus 150 ml Salpetersäure (D1,41) und 175 ml konz. Schwefelsäure zugetropft. Man rührt für 1 Stunde bei 10°C, dann für 1 weitere Stunde bei 20°C nach und erwärmt zum Schluss für 5 Minuten auf 40°C. Der gekühlte Ansatz wird auf 500 g Eis gegossen und die organische Phase mit Methylenchlorid extrahiert. Nach Abdestillieren des Lösungsmittels wird der Rückstand im Vakuum destilliert. Man erhält 198 g 6-Nitro-2,2,3-trifluorbenzodioxen(1,4) vom Siedepunkt 100 bis 102°C/1,33 mbar; $n_D^{20}$ = 1,5078.

In analoger Weise werden die folgenden Nitroverbindungen erhalten:

| Beispiel | Strukturformel | Ausbeute | Siedepunkt | Brechungsindex $n_D^{20}$ |
|---|---|---|---|---|
| (IXb)-2 | 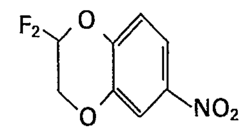 | 91% | 92–93°C/0,27 mbar | 1,534 |
| (IXb)-3 | 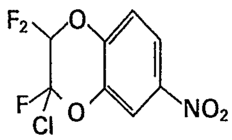 | 93% | 123–125°C/16 mbar | 1,506 |

E) Benzodioxene(1,4) der allgemeinen Formel VIIb und VIIIc:

Beispiel (VIIIb)-1

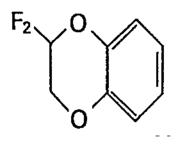

110 g Brenzkatechin werden zusammen mit 70 g Kaliumhydroxid in 300 ml Tetramethylensulfon vorgelegt und unter Rühren innerhalb von 30 Minuten auf 100°C erhitzt. Bei einer Temperatur von 100 bis 110°C werden 140 g 1,1-Difluor-2-chloräthylen eingeleitet (Dauer ca. 3 Stunden). Anschliessend wird bei 20 mbar das Produkt über eine kleine Kolonne in eine gut gekühlte Vorlage destilliert. Man erhitzt hierbei bis zu einer Innentemperatur von 100°C. Der Inhalt der Vorlage wird in einen Scheidetrichter überführt und die organische Phase von der wässrigen abgetrennt. Man erhält 112 g (65% d.Th.) 2,2-Difluor-1,4-benzodioxen mit einem Brechungsindex $n_D^{20}$ = 1,4802, das nach GC-Analyse rein ist.

Beispiel (VIIIb)-2

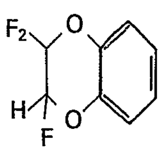

In 600 ml Tetramethylensulfon werden 220 g Brenzkatechin und 130 g Natriumhydroxid bei 95 bis 105°C vorgelegt und bei dieser Temperatur unter Rühren 330 g Trifluorchloräthylen eingeleitet. Man destilliert anschliessend den Ansatz bei 20 mbar über eine Kolonne und fängt eine Fraktion vom Siedepunkt 20 bis 60°C/20 mbar in einer gut gekühlten Vorlage auf. Nachdem die $H_2O$-Phase abgetrennt wurde, verbleiben 332 g reines 2,2,3-Trifluor-1,4-benzodioxen (Ausbeute 87% d.Th.) vom Siedepunkt 54-5°C/16 mbar; $n_D^{20}$ = 1,4525.

Beispiel (VIIIc)

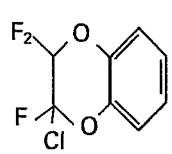

50 g 2,2,3-Trifluorbenzodioxen(1,4) werden in 200 ml Tetrachlorkohlenstoff bei 80°C mit einer UV-Lampe bestrahlt, und innerhalb von 3 Stunden werden 140 g Chlor eingeleitet. Nach Ausblasen von überschüssigem Chlor und Chlorwasserstoff mit Stickstoff wird das Lösungsmittel abdestilliert. Durch Destillation über eine kleine Kolonne erhält man 51 g 2,2,3-Trifluor-3-chlor-benzodioxen(1,4) vom Siedepunkt 57 bis 59°C/17,3 mbar; $n_D^{20}$ = 1,4598.

Beispiel I

Pre-emergence-Test

Lösungsmittel: 5 Gewichtsteile Aceton

Emulgator: 1 Gewichtsteil Alkylarylpolyglycoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Test zeigen die erfindungsgemässen N-Phenylharnstoffe der allgemeinen Formel I sehr gute Wirkung, insbesondere die Wirkstoffe gemäss Herstellungsbeispielen (I)-5, (I)-7, (I)-9, (I)-11.

Beispiel II

Post-emergence-Test

Lösungsmittel: 5 Gewichtsteile Aceton

Emulgator: 1 Gewichtsteil Alkylarylpolyglycoläther

Zur Herstellung einer zweckmässigen Wirkstoffzubereitung vermischt man 1 Gewichtsteil Wirkstoff mit der angegebenen Menge Lösungsmittel,

gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschte Wirkstoffmenge pro Flächeneinheit ausgebracht wird. Die Konzentration der Spritzbrühe wird so gewählt, dass in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Test zeigen die erfindungsgemässen N-Phenylharnstoffe der allgemeinen Formel I sehr gute Wirkung, insbesondere die Wirkstoffe gemäss Herstellungsbeispielen (I)-5, (I)-7, (I)-9, (I)-11.

**Patentansprüche**

1. N-Phenylharnstoffe der allgemeinen Formel I

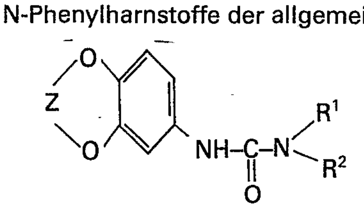

(I),

in welcher

Z für $-CF_2-$, $-CF_2CH_2-$, $-CF_2CHF-$ oder $-CF_2CFCl-$ steht,

$R^1$ für Wasserstoff, $C_{1-4}$-Alkyl, $C_{3-5}$-Alkenyl oder $C_{3-5}$-Alkinyl steht und

$R^2$ für $C_{1-4}$-Alkyl, $C_{3-5}$-Alkenyl, $C_{3-5}$-Alkinyl oder $C_{1-4}$-Alkoxy steht oder

$R^1$ und $R^2$ zusammen mit dem benachbarten N-Atom einen heterocyclischen 5- oder 6-Ring bilden.

2. Verfahren zur Herstellung von N-Phenylharnstoffen der allgemeinen Formel I, dadurch gekennzeichnet, dass man

(a) Isocyanate der allgemeinen Formel II

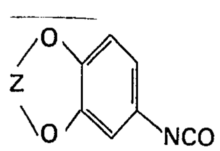

(II),

in welcher

Z die oben angegebene Bedeutung hat,

mit Aminen der allgemeinen Formel III

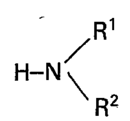

(III),

in welcher

$R^1$ und $R^2$ die oben angegebene Bedeutung haben,

gegebenenfalls in Gegenwart eines Verdünnungsmittels umsetzt oder

(b) für den Fall, dass $R^2$ für $C_{1-4}$-Alkoxy steht, Isocyanate der allgemeinen Formel II mit Hydroxylamin zu Hydroxyharnstoffen der allgemeinen Formel IV umsetzt

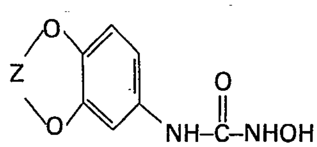

(IV),

in welcher

Z die oben angegebene Bedeutung hat,

und diese anschliessend mit einem Alkylierungsmittel der allgemeinen Formel V

$$X\text{-}R^3 \quad (V),$$

in welcher

$R^3$ für $C_{1-4}$-Alkyl steht und

X für Halogen oder $R^3SO_4$ steht,

in Gegenwart einer Base und gegebenenfalls in Gegenwart eines Verdünnungsmittels umsetzt oder

(c) für den Fall, dass $R^1$ für Wasserstoff und $R^2$ für $C_{1-4}$-Alkyl oder $C_{3-5}$-Alkenyl stehen, Aniline der allgemeinen Formel VI

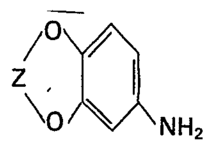

(VI),

in welcher

Z die oben angegebene Bedeutung hat,

mit Isocyanaten der allgemeinen Formel VII

$$R^4\text{–NCO} \quad (VII),$$

in welcher

$R^4$ für $C_{1-4}$-Alkyl oder $C_{3-5}$-Alkenyl steht,

gegebenenfalls in Gegenwart eines Verdünnungsmittels umsetzt.

3. Herbizide Mittel, gekennzeichnet durch einen Gehalt an N-Phenylharnstoffen gemäss Anspruch 1.

4. Verwendung von N-Phenylharnstoffen gemäss Anspruch 1 zur Bekämpfung von Pflanzenwachstum.

5. Verfahren zur Herstellung von herbiziden Mitteln, dadurch gekennzeichnet, dass man N-Phenylharnstoffe gemäss Anspruch 1 mit Streckmitteln und/oder oberflächenaktiven Mitteln vermischt.

6. Isocyanate der allgemeinen Formel II

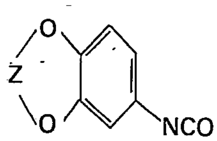

(II),

worin

Z für $-CF_2-$, $-CF_2CH_2-$, $-CF_2CHF-$ oder $-CF_2CFCl-$ steht.

7. Verfahren zur Herstellung von Isocyanaten der allgemeinen Formel II

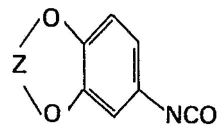

(II),

worin

Z für $-CF_2-$, $-CF_2CH_2-$, $-CF_2CHF-$ oder $-CF_2CFCl-$ steht,

dadurch gekennzeichnet, dass man in einer ersten Stufe Verbindungen der allgemeinen Formel VIII

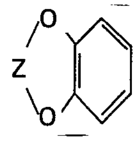

(VIII),

worin

Z die angegebene Bedeutung hat,

in an sich bekannter Weise nitriert, in einer zweiten Stufe die dadurch erhaltenen Nitroverbindungen der allgemeinen Formel IX

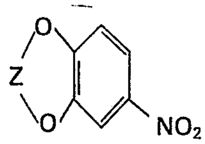

(IX),

worin

Z die angegebene Bedeutung hat,

in an sich bekannter Weise reduziert, vorzugsweise durch katalytische Hydrierung mit Wasserstoff, und in einer dritten Stufe die dadurch erhaltenen Aniline der allgemeinen Formel VI

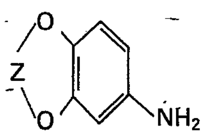

(VI)

worin

Z die angegebene Bedeutung hat,

in an sich bekannter Weise phosgeniert.

**Claims**

1. N-Phenylureas of the general formula I

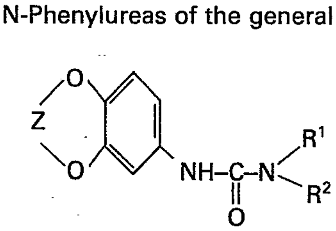

(I),

in which

Z represents $-CF_2-$, $-CF_2CH_2-$, $-CF_2CHF-$ or $-CF_2CFCl-$,

$R^1$ represents hydrogen, $C_{1-4}$-alkyl, $C_{3-5}$-alkenyl or $C_{3-5}$-alkinyl and

$R^2$ represents $C_{1-4}$-alkyl, $C_{3-5}$-alkenyl, $C_{3-5}$-alkinyl or $C_{1-4}$-alkoxy, or

$R^1$ and $R^2$ together with the adjacent N atom form a heterocyclic 5-membered or 6-membered ring.

2. Process for the preparation of N-phenylureas of the general formula I, characterised in that

(a) isocyanates of the general formula II

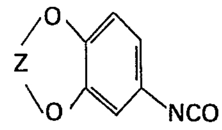

(II),

in which

Z has the meaning indicated above,

are reacted with amines of the general formula III

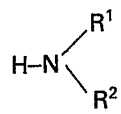

(III),

in which

$R^1$ and $R^2$ have the meaning indicated above,

if appropriate in the presence of a diluent, or

(b) in the case where $R^2$ represents $C_{1-4}$-alkoxy, isocyanates of the general formula II are reacted with hydroxylamine to give hydroxyureas of the general formula IV

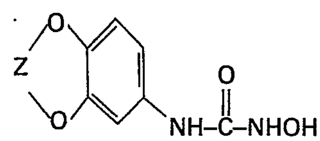

(IV),

in which

Z has the meaning indicated above,

and these hydroxyureas are then reacted with an alkylating agent of the general formula V

$X-R^3$ (V),

in which

$R^3$ represents $C_{1-4}$-alkyl and

X represents halogen or $R^3SO_4$,

in the presence of a base and if appropriate in the presence of a diluent, or

(c) in the case where $R^1$ represents hydrogen and $R^2$ represents $C_{1-4}$-alkyl or $C_{3-5}$-alkenyl, anilines of the general formula VI

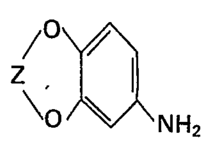

(VI),

in which

Z has the meaning indicated above,

are reacted with isocyanates of the general formula VII

$R^4-NCO$ (VII),

in which

$R^4$ represents $C_{1-4}$-alkyl or $C_{3-5}$-alkenyl,

if appropriate in the presence of a diluent.

3. Herbicidal agents, characterised in that they contain N-phenylureas according to Claim 1.

4. Use of N-phenylureas according to Claim 1 for combating plant growth.

5. Process for the preparation of herbicidal agents, characterised in that N-phenylureas according to Claim 1 are mixed with extenders and/or surface-active agents.

6. Isocyanates of the general formula II

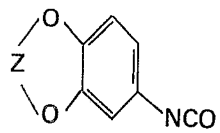

(II),

wherein

Z represents $-CF_2-$, $-CF_2CH_2-$, $-CF_2CHF-$ or $-CF_2CFCl-$.

7. Process for the preparation of isocyanates of the general formfula II

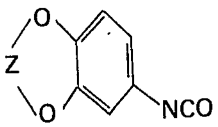

(II),

wherein

Z represents $-CF_2-$, $-CF_2CH_2-$, $-CF_2CHF-$ or $-CF_2CFCl-$,

characterised in that, in a first stage, compounds of the general formula VIII

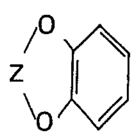

(VIII),

wherein

Z has the meaning indicated,

are nitrated in a manner which is in itself known, the nitro compounds of the general formula IX

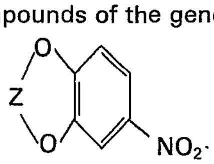

(IX),

wherein

Z has the meaning indicated,

thereby obtained are reduced, in a second stage, in a manner which is in itself known, preferably by catalytic hydrogenation with hydrogen, and, in a third stage, the anilines of the general formula VI

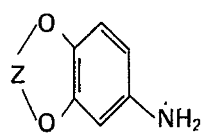

(VI)

wherein

Z has the meaning indicated,

thereby obtained are phosgenated in a manner which is in itself known.

**Revendications**

1. N-phénylurées de formule générale I

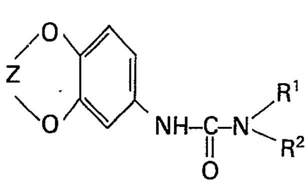

(I),

dans laquelle:

Z représente $-CF_2-$, $-CF_2CH_2-$, $-CF_2CHF-$ ou $-CF_2CFCl-$,

$R^1$ représente l'hydrogène, un groupe alkyle en $C_1$–$C_4$, alcényle en $C_3$–$C_5$ ou alcynyle en $C_3$–$C_5$, et

$R^2$ représente un groupe alkyle en $C_1$–$C_4$, alcényle en $C_3$–$C_5$, alcynyle en $C_3$–$C_5$ ou alcoxy en $C_1$–$C_4$, ou bien

$R^1$ et $R^2$ forment ensemble et avec l'atome d'azote voisin un noyau hétérocyclique à 5 ou 6 chaînons.

2. Procédé de préparation des N-phénylurées de formule générale I, caractérisé en ce que:

(a) on fait réagir des isocyanates de formule générale II

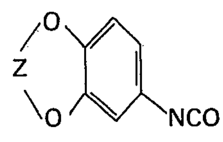

(II),

dans laquelle

Z a la signification indiquée ci-dessus,

avec des amines de formule générale III

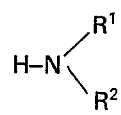

(III),

dans laquelle

$R^1$ et $R^2$ ont les significations indiquées ci-dessus,

éventuellement en présence d'un diluant, ou bien

(b) dans le cas où $R^2$ représente un groupe alcoxy en $C_1$–$C_4$, on fait réagir des isocyanates de formule générale II avec l'hydroxylamine, ce qui donne des hydroxyurées de formule générale IV

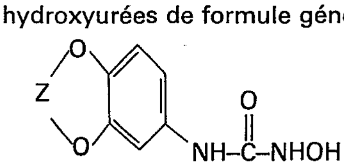

(IV),

dans laquelle

Z a la signification indiquée ci-dessus,

qu'on fait ensuite réagir avec un agent alkylant de formule générale V

$X-R^3$ (V),

dans laquelle

$R^3$ représente un groupe alkyle en $C_1$–$C_4$. et

X représente un halogène ou le groupe $R^3SO_4$,

en présence d'une base et éventuellement en présence d'un diluant, ou bien

(c) dans le cas où $R^1$ représente l'hydrogène et $R^2$ un groupe alkyle en $C_1$–$C_4$ ou alcényle en $C_3$–$C_5$, on fait réagir des anilines de formule générale VI

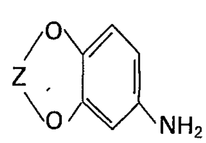

(VI),

dans laquelle
Z a la signification indiquée ci-dessus,
avec des isocyanates de formule générale VII

$R^4$–NCO (VII),

dans laquelle
$R^4$ représente un groupe alkyle en $C_1$–$C_4$ ou alcényle en $C_3$–$C_5$,
éventuellement en présence d'un diluant.

3. Produits herbicides, caractérisés en ce qu'ils contiennent des N-phénylurées selon la revendication 1.

4. Utilisation des N-phénylurées selon la revendication 1 pour combattre la croissance des végétaux.

5. Procédé de préparation de produits herbicides, caractérisé en ce que l'on mélange des N-phénylurées selon la revendication 1 avec des diluants et/ou des agents tensioactifs.

6. Isocyanates de formule générale II

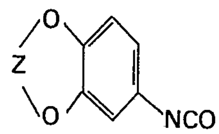
(II),

dans laquelle
Z représente $-CF_2-$, $-CF_2CH_2-$, $-CF_2CHF-$ ou $-CF_2CFCl-$.

7. Procédé de préparation des isocyanates de formule générale II

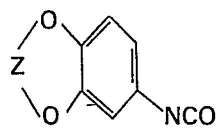
(II),

dans laquelle
Z représente $-CF_2-$, $-CF_2CH_2-$, $-CF_2CHF-$ ou $-CF_2CFCl-$,
caractérisé en ce que, dans un premier stade opératoire, on nitre, de manière connue en soi, des composés de formule générale VIII

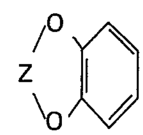
(VIII),

dans laquelle
Z a la signification indiquée ci-dessus,
dans un deuxième stade opératoire, on réduit les composés nitrés obtenus, de formule générale IX

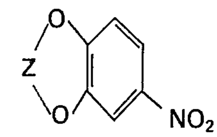
(IX),

dans laquelle
Z a la signification indiquée ci-dessus,
de manière connue en soi, de préférence par hydrogénation catalytique et, dans un troisième stade, on phosgène, de manière connue en soi, les anilines obtenues de formule générale VI

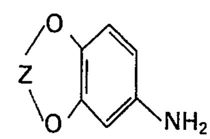
(VI)

dans laquelle
Z a la signification indiquée ci-dessus.